# EUROPÄISCHE PATENTSCHRIFT

(11) **EP 1 136 823 B1**
(45) Veröffentlichungstag und Bekanntmachung des Hinweises auf die Patenterteilung: **01.02.2006**
(21) Anmeldenummer: 01106572.9
(22) Anmeldetag: 15.03.2001
(51) Int. Cl.: G01N 33/68, G01N 1/30

(54) **Verfahren zur Identifizierung und Anreicherung von zellspezifischen Zielstrukturen**
Method for identifying and enriching cell specific target structures
Méthode pour l'identification et l'enrichissement des structures cibles spécifiques aux cellules

(30) Priorität: 24.03.2000 DE 10014708
(43) Veröffentlichungstag der Anmeldung: 26.09.2001
(73) Patentinhaber: MPB MelTec Patent- und Beteiligungsgesellschaft mbH, 39120 Magdeburg (DE)
(72) Erfinder: Schubert, Walter, Dr., 39175 Biederitz (DE)
(74) Vertreter: Schurack, Eduard F.

(56) Entgegenhaltungen:
- EP-A- 0 701 130
- WO-A-93/18068
- WO-A-95/34817
- DE-A- 19 709 348
- US-A- 5 437 987
- WYSOCKI L J ET AL: "PANNING FOR LYMPHOCYTES A METHOD FOR CELL SELECTION" PROCEEDINGS OF THE NATIONAL ACADEMY OF SCIENCES OF THE UNITED STATES, Bd. 75, Nr. 6, 1978, Seiten 2844-2848, XP002901409 1978 EN ISSN: 0027-8424
- SCHUBERT WALTER ET AL: "Detection by 4-parameter microscopic imaging and increase of rare mononuclear blood leukocyte types expressing the Fc-gamma-RIII receptor (CD16) for immunoglobulin G in human sporadic amyotrophic lateral sclerosis (ALS)." NEUROSCIENCE LETTERS, Bd. 198, Nr. 1, 1995, Seiten 29-32, XP000983323 ISSN: 0304-3940

## Beschreibung

Die vorliegenden Erfindung betrifft ein Verfahren zur Identifizierung und Anreicherung von zellspezifischen Zielstrukturen, insbesondere zur Identifikation zellspezifischer Proteinkombinationsmuster auf der Oberfläche von Zellen und zur Anreicherung dieser Zellen.

Die Identifizierung zellspezifischer Zielstrukturen ist von zentraler Bedeutung bei der Aufklärung von Zell-Zell-Interaktionen, die unzählige Wirkungen innerhalb eines Organismus nach sich ziehen können. Insbesondere die Kenntnis krankheitsspezifische Zielstrukturen ist eine entscheidende Voraussetzung für die Entwicklung wirksamer und gleichzeitig nebenwirkungsarmer Arzneimittel.

Es ist bekannt, daß Immunzellen (Lymphozyten) spezifische Kombinationen von Proteinen, auch Proteinkombinationsmuster bzw. kurz PKM genannt, exprimieren, die für eine Bindung an Endothelzellen der Blutgefäße von Gehirn und Muskelgewebe verantwortlich sind. Andere Kombinationen von Proteinen führen dagegen nicht zu einer Bindung an diese Endothelzellen. Überraschenderweise sind diese spezifischen Kombinationen interindividuell konstant und weisen immer dieselben Bindungsfunktionen auf. Es scheint sich folglich bei den spezifischen Proteinkombinationsmustem um einen interindividuellen konstanten Lymphozyten Bindungs-Code der Zelloberfläche für organspezifische Endothelzelloberflächen zu handeln, der eine zellspezifische Zielstruktur darstellt. Zellspezifische Zielstrukturen können folglich ganz spezifische Proteinkombinationsmuster aufweisen.

Auch invasive Tumorzellen verfügen über spezifische Proteinkombinationsmuster an ihren Zelloberflächen, die zu einem gezielten, d.h. organselektiven Invasionsverhalten führen. Solche Proteinkombinationsmuster stellen daher Zielstrukturen für mögliche Arzneimittel dar.

Unbedingte Voraussetzung für die Entwicklung solcher hoch-selektiver Arzneimittel ist jedoch die Kenntnis der molekularen Zusammensetzung dieser Zielstrukturen.

Aus dem Stand der Technik sind Verfahren zur Identifizierung von Zielstrukturen bekannt, die auf der Analyse von Gen-Expressionsprofilen von kranken Geweben oder Zellen im Vergleich zu Gen-Expressionsprofilen von gesunden Geweben oder Zellen beruhen, wobei sowohl Proteinexpressionsprofile als auch Expressionsprofile der Messenger Ribonukleinsäure (mRNA) Aufschluß über das Auftreten neuer Proteine, fehlregulierter oder abnorm modifizierter Proteine in kranken Geweben oder Zellen geben sollen (z.B. in: F. Lottspeich/H.Zorbas; Bioanalytik; Spektrum Akademischer Verlag; Heidelberg, 1998).

Diese Verfahren gehen jedoch alle von Zellhomogenaten aus, denen in der Regel Tausende oder Millionen von Zellen zugrunde liegen, da nur mit Hilfe dieser Zellmengen Expressionsprofile der oben genannten Art erstellt werden können. In den Zellhomogenaten liegen die Zellen in aufgeschlossener Form vor, damit die Proteine oder mRNA-Moleküle mit Hilfe biochemischer Verfahren extrahiert und getrennt werden können.

WO-A-95/34817 und EP-A-0 701 130 offenbaren Verfahren zum Auswahl bestimmter Zellen aus einem Zellgemisch. Bei diesen Verfahren wird vorausgestetz, dass die Zielstrukture an der Oberfläche der Partikeln, die mit den heterogenen Zellengemisch reagieren, bereits bekannt sind. Somit können solche Verfahren zur Identifizierung/Selektion von Zellen erfolgreich angewendet werden.

Diese bekannten Verfahren sind nicht dazu geeignet, Proteinkombinationsmuster zu identifizieren, da die einzelnen Proteinkomponenten eines solchen Proteinkombinationsmusters durch die Erzeugung von Zellhomogenaten und durch die darauffolgenden Extraktionsvorgänge vollständig getrennt werden und die wesentliche Information bezüglich ihrer zell- und gewebetopologischen Lage verloren geht. Durch die Zerstörung der Zellkompartimente können weiterhin keine Informationen bezüglich der Kombinationen von Proteinen innerhalb dieser Zellkompartimente und ihre relative topologische Beziehung zueinander gewonnen werden.

Ein weiterer Nachteil der bekannten Verfahren besteht darin, daß die Präparationsschritte des Gewebes oder der Zellen von ihrer Entnahme oder Gewinnung bis zum Schritt der Isolierung bzw. Auftrennung von Proteinen einer großen Zahl von variablen externen Einflüssen unterliegen können, die nur schwer kontrollierbar und zu vereinheitlichen sind.

Ein weiterer Nachteil der bekannten Verfahren ist außerdem, daß keine Analytik auf dem Einzelzellniveau durchführbar ist, so daß Unterschiede der einzelnen Zellen bezüglich ihrer Proteinkombinationsmuster nicht erfaßbar sind. Außerdem können Proteine, die nur in geringer Menge vorliegen, durch die bekannten Verfahren nicht delektiert werden. Dies betrifft insbesondere Proteine oder spezifische Proteinkombinationen, die beispielsweise nur in wenigen, jedoch pathogenen, krankheitsspezifischen Zellen vorkommmen.

Aufgabe der Erfindung ist es daher ein Verfahren der oben genannten Art bereitzustellen, durch das zellspezifische Proteinkombinationsmuster identifiziert und angereichert werden können und durch das die aufgeführten Nachteile des Stands der Technik überwunden werden.

Diese Aufgabe wird gelöst durch ein erfindungsgemäßes Verfahren zur Identifizierung und Anreicherung von zellspezifischen Zielstrukturen, insbesondere zur Identifikation zellspezifischer Proteinkombinationsmuster auf der Oberfläche von Zellen und zur Anreicherung dieser Zellen mit den Merkmalen des Anspruchs 1.

Vorteilhafte Ausgestaltungen des erfindungsgemäßen Verfahrens sind in den Unteransprüchen beschrieben.

Ein erfindungsgemäßes Verfahren zur Identifizierung und Anreicherung von zellspezifischen Zielstrukturen, insbesondere zur Identifikation zellspezifischer Proteinkombinationsmuster auf der Oberfläche von Zellen und zur Anreicherung dieser Zellen, umfaßt die Verfahrensschritte: (a) Aufbringen eines heterogenen Zellgemisches auf eine oder mehrere Oberflächen mit vordefinierten Strukturen, wobei Zellen mit entsprechenden Zielstrukturen an die Oberfläche oder die Oberflächen gebunden werden; (b) Abführen der nicht-bindenden Zellen des Zellgemisches von der oder den Oberflächen; (c) Identifizierung der zellspezifischen Zielstrukturen, die für die Bindung der Zellen an der oder den Oberflächen verantwortlich sind; (d) Auswahl und Anreicherung von Zellen mit identischen zellspezifischen Zielstrukturen auf der oder den Oberflächen; und (e) biochemische Charakterisierung der gemäß Verfahrensschritt (d) ausgewählten Zielstrukturen. Dadurch ist es möglich, daß diejenigen zellspezifischen Zielstrukturen, insbesondere Proteinkombinationen der Oberfläche von Zellen, identifiziert werden können, die für die Bindung an vordefinierte Strukturen verantwortlich sind. Zudem können diese zellspezifischen Zielstrukturen wie z.B. einheitliche Zelloberflächen angereichert werden, um sie dann biochemischen Analyseverfahren zuzuführen. So können Proteine, die nur in geringer Menge vorliegen, angereichert und analysiert werden. Dies betrifft insbesondere Proteine oder spezifische Proteinkombinationen, die beispielsweise nur in wenigen, jedoch pathogenen, krankheitsspezifischen Zellen vorkommmen. Auf diese Weise werden also Zellen ein- und desselben Funktionstyps in Bezug auf hochselektive Bindung für weitergehende Analysen der von ihnen exprimierten Proteine bereitgestellt. Vorteilhafterweise erfolgt die biochemische Charakterisierung der ausgewählten und angereicherten Zielstrukturen gemäß Verfahrensschritt e) mittels eines Molekül- oder Molekülkomplextrennverfahrens, wie z.B. eines Proteintrennverfahrens, insbesondere eine 2D-Gel-Elektrophorese.

Ein Ausführungsbeispiel für die Durchführung der Verfahrensschritte (a) bis (b) der Erfindung beinhaltet folgendes Teilverfahren: 1) Ein- bzw. Aufbringen des flüssigen, heterogen Zellgemisches in einen Kanal einer Analysevorrichtung und auf einen Objektträger mit einer fixierten, eine vordefinierte Struktur enthaltene Oberfläche; 2) Entfernung der nicht-bindenden Zellen über eine Kanalöffnung des Kanals und Auffangen dieses Materials in einem Gefäß; 3) Kühlung des Objektträgers durch einen Objektträger-Thermostat, und 4) Fixierung der auf dem Objektträger gebundenen Zellen.

In einer vorteilhaften Ausgestaltung des erfindungsgemäßen Verfahrens wird das heterogene Zellgemisch aus menschlichen oder tierischen Gewebe oder menschlichen oder tierischen Körperflüssigkeiten isoliert oder es sind angezüchtete Zellen und die Oberfläche ist ein menschlicher oder tierischer Gewebeschnitt und/oder Endothelzellen und/oder Proteinchips und/oder ein angezüchtetes menschliches oder tierisches Gewebestück. Dadurch ist eine rasche und zielsichere Identifikation der zellspezifischen Zielstrukturen für die Entwicklung von Arzneimitteln gewährleistet.

In einer weiteren vorteilhaften Ausgestaltung des erfindungsgemäßen Verfahrens wird die Identifizierung der zellspezifischen Zielstrukturen mit einem Verfahren durchgeführt, das folgende Schritte umfaßt: (I) Automatisiertes Aufbringen einer Reagenzlösung Y1, die mindestens ein Markiermolekül aufweist, auf die zellspezifische Zielstruktur; (II) Einwirken der Reagenzlösung Y1 und automatisierte Detektion mindestens eines Markierungsmusters der mit der Reagenzlösung Y1 markierten Zielstruktur; (III) Entfernen der Reagenzlösung Y1 vor oder nach der Detektion des Markierungsmusters und Wiederholung der Schritte (I) und (II) mit weiteren Reagenzlösungen Yn (n= 2, 3...N), die jeweils das mindestens eine Markiermolekül und/oder mindestens ein anderes Markiermolekül aufweisen; und (IV) Zusammenfassen der jeweils in Schritt II) detektierten Markierungsmuster zu einem komplexen molekularen Kombinationsmuster der zellspezifischen Zielstruktur. Mit diesem Identifikationsverfahren ist eine schnelle und zielsichere Identifikation von zellspezifischen Zielstrukturen wie z.B. Proteinkombinationsmuster auf Zelloberflächen gewährleistet.

In einer weiteren vorteilhaften Ausgestaltung des erfindungsgemäßen Verfahrens wird nach dem Verfahrensschritt (d) folgender Verfahrensschritt durchgeführt: (d1) Durchführung von Inhibitionsexperimenten bezüglich eines oder mehrerer Bestandteile der in Verfahrensschritt (d) ausgewählten zellspezifischen Zielstrukturen zur Erkennung einer Bindungshierarchie der Bestandteile, wobei die Bestandteile einzelne oder mehrere Proteine eines zellspezifischen Proteinkombinationsmusters sind. Dadurch kann ermittelt werden, welche Proteine für diese selektive Bindung in der Proteinhierarchie besonders relevant sind und welche nicht. Relevante Proteine sind Targets für die Entwicklung hoch-selektiver Arzneimittel.

In einer weiteren vorteilhaften Ausgestaltung des erfindungsgemäßen Verfahrens werden anstelle von Verfahrensschritt (e) folgende Verfahrensschritte durchgeführt: (f) Automatisiertes Aufbringen einer Reagenzlösung Y1, die mindestens ein Markiermolekül aufweist, auf die ausgewählte und angereicherte zellspezifische Zielstruktur; (g) Einwirken der Reagenzlösung Y1 und automatisierte Detektion mindestens eines Markierungsmusters der mit der Reagenzlösung Y1 markierten Zielstruktur; (h) Entfernen der Reagenzlösung Y1 vor oder nach der Detektion des Markierungsmusters und Wiederholung der Schritte (f) und (g) mit weiteren Reagenzlösungen Yn (n= 2, 3...N), die jeweils das mindestens eine Markiermolekül und/oder mindestens ein anderes Markiermolekül aufweisen; und (i) Zusammenfassen der jeweils in Schritt (g) detektierten Markierungsmuster zu einem komplexen molekularen Kombinationsmuster der ausgewählten und angereicherten zellspezifischen Zielstruktur. Erfindungsgemäß angereicherte und ausgewählte Zellen desselben Typs erlauben es nunmehr, daß mit Hilfe der Verfahrensschritte (f) bis (i) die zellspezifischen Zielstrukturen noch wesentlich genauer untersucht werden können.

Zusammenfassend ermöglicht es das erfindungsgemäße Verfahren, daß unter anderem für die Arzneimittelentwicklung relevante valide Zielstrukturen effizient gefunden werden können, indem biologische Selektivitätsmechanimen für die Identifikation von Zielstrukturen zugrunde gelegt werden.

## Patentansprüche

1. In vitro Verfahren zur Identifizierung und Anreicherung von zellspezifischen Zielstrukturen, insbesondere zur Identifikation zellspezifischer Proteinkombinationsmuster auf der Oberfläche von Zellen und zur Anreicherung dieser Zellen, wobei das Verfahren folgende Schritte umfaßt:
a) Aufbringen eines heterogenen Zellgemisches auf eine oder mehrere Oberflächen eines menschlichen oder tierischen Gewebeschnitts und/oder von Endothelzellen und/oder eines angezüchtetes menschliches oder tierisches Gewebestücks, wobei Zellen mit entsprechenden Zielstrukturen an die Oberfläche oder die Oberflächen gebunden werden;
b) Abführen der nicht-bindenden Zellen des Zellgemisches von der oder den Oberflächen;
c) Identifizierung der zellspezifischen Zielstrukturen, die für die Bindung der Zellen an der oder den Oberflächen verantwortlich sind;
d) Auswahl und Anreicherung von Zellen mit identischen zellspezifischen Zielstrukturen auf der oder den Oberflächen; und
e) biochemische Charakterisierung der gemäß Verfahrensschritt d) ausgewählten Zielstrukturen.

2. Verfahren nach Anspruch 1,
**dadurch gekennzeichnet,**
**daß** das heterogene Zellgemisch aus menschlichen oder tierischen Gewebe oder menschlichen oder tierischen Körperflüssigkeiten isoliert wird oder angezüchtete Zellen sind.

3. Verfahren nach einem der vorhergehenden Ansprüche,
**dadurch gekennzeichnet,**
**daß** die Identifizierung der zellspezifischen Zielstrukturen mit einem Verfahren durchgeführt wird, daß folgende Schritte umfaßt:
I) Automatisiertes Aufbringen einer Reagenzlösung Y1, die mindestens ein Markiermolekül aufweist, auf die zellspezifische Zielstruktur;
II) Einwirken der Reagenzlösung Y1 und automatisierte Detektion mindestens eines Markierungsmusters der mit der Reagenzlösung Y1 markierten Zielstruktur;
III) Entfernen der Reagenzlösung Y1 vor oder nach der Detektion des Markierungsmusters und Wiederholung der Schritte I) und II) mit weiteren Reagenzlösungen Yn (n= 2, 3...N), die jeweils das mindestens eine Markiermolekül und/oder mindestens ein anderes Markiermolekül aufweisen; und
IV) Zusammenfassen der jeweils in Schritt II) detektierten Markierungsmuster zu einem komplexen molekularen Kombinationsmuster der zellspezifischen Zielstruktur.

4. Verfahren nach einem der vorhergehenden Ansprüche,
**dadurch gekennzeichnet,**
**daß** die biochemische Charakterisierung der ausgewählten Zielstrukturen gemäß Verfahrensschritt e) mittels eines Molekül- oder Molekülkomplextrennverfahrens, insbesondere Proteintrennverfahrens, erfolgt.

5. Verfahren nach Anspruch 4,
**dadurch gekennzeichnet,**
**daß** das Proteintrennverfahren eine 2D-Gel-Elektrophorese ist.

6. Verfahren nach einem der vorhergehenden Ansprüche,
**dadurch gekennzeichnet,**
**daß** nach dem Verfahrensschritt d) folgender Verfahrensschritt durchgeführt wird:
d1) Durchführung von Inhibitionsexperimenten bezüglich eines oder mehrerer Bestandteile der in Verfahrensschritt d) ausgewählten zellspezifischen Zielstrukturen zur Erkennung einer Bindungshierarchie der Bestandteile.

7. Verfahren nach Anspruch 6,
**dadurch gekennzeichnet,**
**daß** die Bestandteile einzelne oder mehrere Proteine eines zellspezifischen Proteinkombinationsmusters sind.

8. Verfahren nach Anspruch 1,
**dadurch gekennzeichnet,**
**daß** anstelle von Verfahrensschritt e) folgende Verfahrensschritte durchgeführt werden:
f) Automatisiertes Aufbringen einer Reagenzlösung Y1, die mindestens ein Markiermolekül aufweist, auf die ausgewählte und angereicherte zellspezifische Zielstruktur;
g) Einwirken der Reagenzlösung Y1 und automatisierte Detektion mindestens eines Markierungsmusters der mit der Reagenzlösung Y1 markierten Zielstruktur;
h) Entfernen der Reagenzlösung Y1 vor oder nach der Detektion des Markierungsmusters und Wiederholung der Schritte f) und g) mit weiteren Reagenzlösungen Yn (n= 2, 3...N), die jeweils das mindestens eine Markiermolekül und/oder mindestens ein anderes Markiermolekül aufweisen; und
i) Zusammenfassen der jeweils in Schritt g) detektierten Markierungsmuster zu einem komplexen molekularen Kombinationsmuster der ausgewählten und angereicherten zellspezifischen Zielstruktur.

## Claims

1. In vitro method for identifying and enriching cell-specific target structures, especially for identifying cell-specific protein combination patterns on the surface of cells, and for enriching these cells, the method including the following steps:
a) applying a heterogeneous cell mixture onto one or plural surfaces of a human or animal tissue section and/or endothelial cells and/or a grown human or animal tissue piece, wherein cells having corresponding target structures are bound to the surface or the surfaces;
b) removing the non-binding cells of the cell mixture from the surface or surfaces;
c) identifying the cell-specific target structures, which are responsible for binding the cells to the surface or surfaces;
d) selecting and enriching cells with identical cell-specific target structures on the surface or surfaces; and
e) biochemically characterizing the target structures selected according to method step d).

2. Method according to claim 1,
**characterized in that**
the heterogeneous cell mixture is isolated from human or animal tissue or human or animal body fluids or is grown cells.

3. Method according to any one of the preceding claims,
**characterized in that**
the identification of the cell-specific target structures is performed with a method including the following steps:
I) automated application of a reagent solution Y1 having at least one marker molecule to the cell-specific target structure;
II) reacting the reagent solution Y1 and automated detection of at least one marking pattern of the target structure marked with the reagent solution Y1;
III) removing the reagent solution Y1 before or after detecting the marking pattern, and repeating steps I) and II) with further regent solutions Yn (n = 2, 3 ...N) each having the at least one marker molecule and/or at least another marker molecule; and
IV) combining the marking patterns each detected in step II) to a complex molecular combination pattern of the cell-specific target structure.

4. Method according to any one of the preceding claims,
**characterized in that**
the biochemical characterization of the selected target structures according to method step e) is effected by means of a molecule or molecule-complex separation method, especially a protein separation method.

5. Method according to claim 4,
**characterized in that**
the protein separation method is 2D gel electrophoresis.

6. Method according to any one of the preceding claims,
**characterized in that**
after method step d), the following method step is performed:
d1) performing inhibition experiments with respect to one or more components of the cell-specific target structures selected in method step d) for recognizing a binding hierarchy of the components.

7. Method according to claim 6,
**characterized in that**
the components are individual or plural proteins of a cell-specific protein combination pattern.

8. Method according to claim 1,
**characterized in that**
instead of method step e), the following method steps are performed:
f) automated application of a reagent solution Y1 having at least one marker molecule to the selected and enriched cell-specific target structure;
g) reacting the reagent solution Y1 and automated detection of at least one marking pattern of the target structure marked with the reagent solution Y1; h) removing the reagent solution Y1 before or after detection of the marking pattern, and repeating the steps f) and g) with further reagent solutions Yn (n = 2, 3 ... N) each having the at least one marker molecule and/or at least another marker molecule; and
i) combining the marking patterns each detected in step g) to a complex molecular combination pattern of the selected and enriched cell-specific target structure.

## Revendications

1. Procédé in vitro d'identification et d'enrichissement de structures cibles spécifiques aux cellules, en particulier, d'identification de motifs de combinaison de protéines à la surface de cellules et d'enrichissement de ces cellules, le procédé comprenant les étapes suivantes :
a) dépôt d'un mélange hétérogène de cellules sur une ou plusieurs surfaces d'une coupe de tissu humain ou animal et/ou de cellules endothéliales et/ou d'une partie cultivée de tissu humain ou animal, des cellules de structures cible correspondantes étant liées à la surface ou aux surfaces ;
b) évacuation des cellules non liantes du mélange de cellules de la ou des surfaces ;
c) identification des structures cibles spécifiques aux cellules qui sont responsables de la liaison des cellules à la ou les surfaces ;
d) sélection et enrichissement de cellules de structures cibles spécifiques aux cellules identiques sur la ou les surface ; et
e) caractérisation biochimique des structures cibles sélectionnées selon l'étape d).

2. Procédé selon la revendication 1,
**caractérisé en ce que**
le mélange hétérogène de cellules est isolé ou est des cellules cultivées de tissus humains ou animaux ou de fluides corporels humains ou animaux.

3. Procédé selon l'une quelconque des revendications précédentes,
**caractérisé en ce que**
l'identification des structures cibles spécifiques aux cellules s'effectue par un procédé qui comprend les étapes suivantes :
I) dépôt automatisé d'une solution de réactif Y1, qui présente au moins une molécule de marquage, sur la structure cible spécifique aux cellules ;
II) réaction de la solution de réactif Y1 et détection automatisée d'au moins un motif de marquage de la structure cible marquée par la solution de réactif Y1 ;
III) élimination de la solution de réactif Y1 avant ou après la détection du motif de marquage et répétition des étapes I) et II) avec d'autres solutions de réactif Yn (n = 1, 2, ... N) qui présentent chaque fois la au moins une molécule de marquage et/ou au moins une autre molécule de marquage ; et
IV) regroupement des motifs de marquage chaque fois détectés à l'étape II) en un motif combiné moléculaire complexe de la structure cible spécifique aux cellules.

4. Procédé selon l'une quelconque des revendications précédentes,
**caractérisé en ce que**
la caractérisation biochimique des structures cibles sélectionnées selon l'étape de procédé e) s'effectue au moyen d'un procédé de séparation de molécule ou de complexe de molécules, en particulier d'un procédé de séparation de protéines.

5. Procédé selon la revendication 4,
**caractérisé en ce que**
le procédé de séparation de protéines est une électrophorèse en gel 2D.

6. Procédé selon l'une quelconque des revendications précédentes,
**caractérisé en ce que**
après l'étape de procédé d), l'étape de procédé suivante est exécutée :
d1) exécution d'expériences d'inhibition en rapport avec un ou plusieurs composants des structures cible spécifiques aux cellules sélectionnées à l'étape de procédé d) pour distinguer la hiérarchie de liaison des composants.

7. Procédé selon la revendication 6,
**caractérisé en ce que**
les composants sont des protéines isolées ou plusieurs protéines d'un motif de combinaison de protéines spécifique aux cellules.

8. Procédé selon la revendication 1
**caractérisé en ce que**
à la place de l'étape de procédé e), les étapes de procédé suivantes sont exécutées :
f) dépôt automatisé d'une solution de réactif Y1, qui présente au moins une molécule de marquage, sur la structure cible spécifique aux cellules sélectionnée et enrichie ;
g) réaction de la solution de réactif Y1 et détection automatisée d'au moins un motif de marquage de la structure cible marquée par la solution de réactif Y1;
h) élimination de la solution de réactif Y1 avant ou après la détection du motif de marquage et répétition des étapes f) et g) avec d'autres solutions de réactif Yn (n = 1, 2, ... N) qui présentent chaque fois la au moins une molécule de marquage et/ou au moins une autre molécule de marquage ; et
i) regroupement des motifs de marquage chaque fois détectés à l'étape g) en un motif combiné moléculaire complexe de la structure cible spécifique aux cellules sélectionnée et enrichie.
